Europäisches Patentamt

European Patent Office

Office européen des brevets

(11) Veröffentlichungsnummer : **0 349 797 B1**

# (12) EUROPÄISCHE PATENTSCHRIFT

(45) Veröffentlichungstag der Patentschrift :
30.09.92 Patentblatt 92/40

(51) Int. Cl.⁵ : **A61K 31/195, A61K 47/12**

(21) Anmeldenummer : **89110619.7**

(22) Anmeldetag : **12.06.89**

(54) **Stabilisierte, N-acetylcysteinhaltige Arzneimittelzubereitung.**

(30) Priorität : **30.06.88 DE 3822096**

(43) Veröffentlichungstag der Anmeldung :
**10.01.90 Patentblatt 90/02**

(45) Bekanntmachung des Hinweises auf die
Patenterteilung :
**30.09.92 Patentblatt 92/40**

(84) Benannte Vertragsstaaten :
**AT BE CH DE ES FR GB GR IT LI LU NL SE**

(56) Entgegenhaltungen :
**CHEMICAL ABSTRACTS, Band 104, Nr. 12, 24
März 1986, Columbus, OH (US); C.STRUHAR et
al., Seite 438, Nr. 95381c.
CHEMICAL ABSTRACTS, Band 96, Nr. 9, 01
März 1982, Columbus, OH (US); R.R.PFISTER
et al., Seite 62, Nr. 62950a.**

(56) Entgegenhaltungen :
**CHEMICAL ABSTRACTS, Band 84, Nr. 20, 17
Mai 1976, Columbus, OH (US); Seite 358, Nr.
140738f.
INVEST. OPHTALMOL. VIS. SCI., Band 25, Nr.
3, September 1981; Seiten 486-490.**

(73) Patentinhaber : **Klinge Pharma GmbH
Berg-am-Laim-Strasse 129
W-8000 München 80 (DE)**

(72) Erfinder : **Stanislaus, Fritz, Dr.
Halserspitzstrasse 12
W-8000 München 80 (DE)**
Erfinder : **Klemm, Fritz-Heinrich
Phantasiestrasse 5
W-8000 München 82 (DE)**

(74) Vertreter : **Kolb, Helga, Dr. Dipl.-Chem. et al
Hoffmann, Eitle & Partner Patentanwälte
Arabellastrasse 4
W-8000 München 81 (DE)**

EP 0 349 797 B1

## Beschreibung

Die Erfindung betrifft eine stabilisierte Arzneimittelzubereitung, insbesondere ein stabilisiertes N-Acetylcystein-haltiges Arzneimittel sowie die Verwendung von Ascorbinsäure bzw. Ascorbat zur Stabilisierung desselben.

N-Acetylcystein zeigt bei verschiedenen Erkrankungen der Lunge und der Bronchien eine schleimlösende Wirkung. Das acetylierte Cystein zeichnet sich gegenüber der Ausgangssubstanz Cystein im Körper durch erhöhte Stabilität aus. Dies ist darauf zurückzuführen, dass der oral aufgenommene Wirkstoff nicht nur als freie Substanz, sondern darüber hinaus auch über Disulfidbrücken reversibel an Protein gebunden oder fest in Proteine inkorporiert vorliegt. Die dabei bestehende labile Disulfidbindung zwischen N-Acetylcystein und Proteinen kann daher als Schutzform angesehen werden.

Einen derartigen Schutz geniesst jedoch der Wirkstoff in wässriger Lösung oder Dispersion nicht, insbesondere dann nicht, wenn entsprechend der Empfehlung in der Arzneimittelpackungsbeilage das im Handel erhältliche Granulat zur Einnahme in Wasser, Tee oder Fruchtsäften aufgelöst wird. Dann besteht die Gefahr, dass, besonders bei Auflösung in einem warmen Medium, eine Zersetzung des Wirkstoffes erfolgt. Das Ausmass der Zersetzung ist dann besonders stark, wenn der Wirkstoff nach Auflösung in z.B. warmem Wasser oder Tee nicht gleich eingenommen, sondern einige Zeit stehen gelassen wird. So konnte z.B. festgestellt werden, dass beim Auflösen eines N-Acetyl-haltigen Arzneimittels in auf ca. 70°C erwärmtem Tee unter Bedingungen, wie sie in der Thermoskanne, der Thermosflasche oder auf einer Warmhalteplatte gegeben sind, nach ca. 1 h eine nennenswerte Menge des Wirkstoffes zersetzt ist.

Es ist bekannt, wässrige Ascorbinsäurelösungen durch Zugabe geringer Gewichtsmengen einer Thioverbindung zu stabilisieren. So beschreibt JP 49/92219 die Stabilisierung von wässrigen Ascorbinsäurelösungen durch Zugabe von nicht-therapeutischen Mengen an N-Acetylcystein und Sulfit.

In der Publikation "Stabilisation of solutions for injections with vitamin C" in Farm. Obz., 54(11),513-18, wird die Stabilisierung von 10%igen L-Ascorbinsäurelösungen durch Zugabe des Chelatbildners HEDTA und N-Acetylcystein beschrieben.

Eine Stabilisierung von wässrigen N-Acetylcysteinlösungen durch Ascorbinsäure ist aus dem vorstehend genannten Stand der Technik, insbesondere unter Berücksichtigung der jeweiligen Oxidationspotentiale von Ascorbinsäure und N-Acetylcystein nicht abzuleiten.

So weist Ascorbinsäure ein Standard-Oxidationspotential von -0,115 Volt bei pH 5,2 (30°C) und von -0,140 Volt bei pH 4,7 (30°C) auf (Stewart, P.J., Tucker, I.G., Aust. J., Hosp. Pharm., 15 (2), 1985, Seite 111 ff.). Es ist anzunehmen, dass der Oxidationsmechanismus von N-Acetylcystein demjenigen von Cystein vergleichbar ist und somit für N-Acetylcystein ein Standard-Oxidationspotential ähnlich dem von Cystein angenommen werden kann. Cystein weist ein Standard-Oxidationspotential von +0,220 Volt (25°C) auf (siehe vorstehende Veröffentlichung von P.J. Stewart et al.).

Um einen Wirkstoff vor Oxidation durch Zugabe eines Hilfsstoffes zu schützen, sollte letzterer ein höheres Redoxpotential als der zu schützende Stoff aufweisen. Dies trifft theoretisch und praktisch für die aus dem Stand der Technik bekannte Stabilisierung von Ascorbinsäure durch N-Acetylcystein zu, jedoch nicht für den umgekehrten Fall, nämlich die Stabilisierung von N-Acetylcystein durch Ascorbinsäure.

Aufgabe der vorliegenden Erfindung ist es, ein stabilisiertes N-Acetylcystein-haltiges Arzneimittel zur Verfügung zu stellen.

Die vorstehende Aufgabe wird gemäss der Aufgabe dadurch gelöst, dass dem N-Acetylcystein-haltigen Arzneimittel Ascorbinsäure bzw. Ascorbat zugegeben wird.

Das zu stabilisierende N-Acetylcystein-haltige Arzneimittel liegt gemäss der Erfindung in fester Form oder als wässrige Lösung oder Dispersion vor, und wird zur Anwendung am Menschen bevorzugt in wässrige Lösung gebracht. Es ist bevorzugt, das N-Acetylcystein-haltige Arzneimittel zur Einnahme in einem warmen Medium aufzulösen, wobei es in der erfindungsgemäss stabilisierten Form auch beim Stehenlassen über einen längeren Zeitraum seine Wirksamkeit im wesentlichen beibehält.

Die zur Stabilisierung zugegebenen Gewichtsmengen an Ascorbinsäure oder einem Salz derselben können im Bereich von 5 bis 100 Gewichtsteilen, bevorzugt von 10 bis 50 Gewichtsteilen, jeweils bezogen auf 100 Gewichtsteile N-Acetylcystein, variieren. Besonders bevorzugt beträgt die Menge an Ascorbinsäure oder Ascorbat 25 Gewichtsteile pro 100 Gewichtsteile N-Acetylcystein.

Die Erfindung umfasst auch die Verwendung von Ascorbinsäure bzw. einem Salz derselben zur Stabilisierung von N-Acetyl-haltigen Arzneimitteln.

Die gemäss der Erfindung erzielte Schutzwirkung auf N-Acetylcystein ist insofern überraschend, als dieselbe aufgrund der Oxidationspotentiale von N-Acetylcystein einerseits und Ascorbinsäure andererseits nicht zu erwarten war.

Der erfindungsgemässe Stabilisierungseffekt zeigt sich allgemein, wenn N-Acetylcystein in einem wässrigen Medium aufgelöst und dabei einige Zeit stehen gelassen wird. Besonders ausgeprägt ist dieser Effekt beim Auflösen des Arzneimittels in warmem Tee; in diesem Fall wird die Zersetzung des Wirkstoffes, die durch die Anwesenheit von Tee-Inhaltsstoffen, wie Flavonen, Gerbstoffen, etc., noch beschleunigt wird, weitgehend verhindert. Somit wird aufgrund der stabilisierenden Wirkung der Ascorbinsäure die Zersetzung von N-Acetylcystein wesentlich reduziert. Dieser Effekt ist umso vorteilhafter, als die physiologische Verträglichkeit und Unbedenklichkeit von Ascorbinsäure nicht bestritten werden kann, ein Umstand, der nicht für alle Antioxidantien zutrifft.

Im folgenden werden Versuche beschrieben, die den stabilisierenden Effekt auf N-Acetylcystein durch Vitamin-C-Zusatz zeigen.

Vergleichsversuche mit und ohne Zugabe von Ascorbinsäure

Um die stabilisierende Wirkung von Ascorbinsäure auf N-Acetylcystein bei verschiedenen Konzentrationen zu zeigen, wurden jeweils einer Probe Leitungswasser bzw. Pfefferminztee (150 ml), die konstant auf einer Temperatur von 50 bzw. 70°C gehalten wurde, 100 mg N-Acetylcystein sowie jeweils 0, 10, 25, 50 bzw. 100 mg Ascorbinsäure zugegeben. Der Wirkstoff N-Acetylcystein (0,1 g) wurde als Granulat in 2,9 g Saccharose zugegeben.

Die Proben wurden über 4 h auf der genannten Temperatur gehalten, wobei nach 2 und 4 h der noch vorhandene Gehalt an N-Acetylcystein bestimmt wurde.

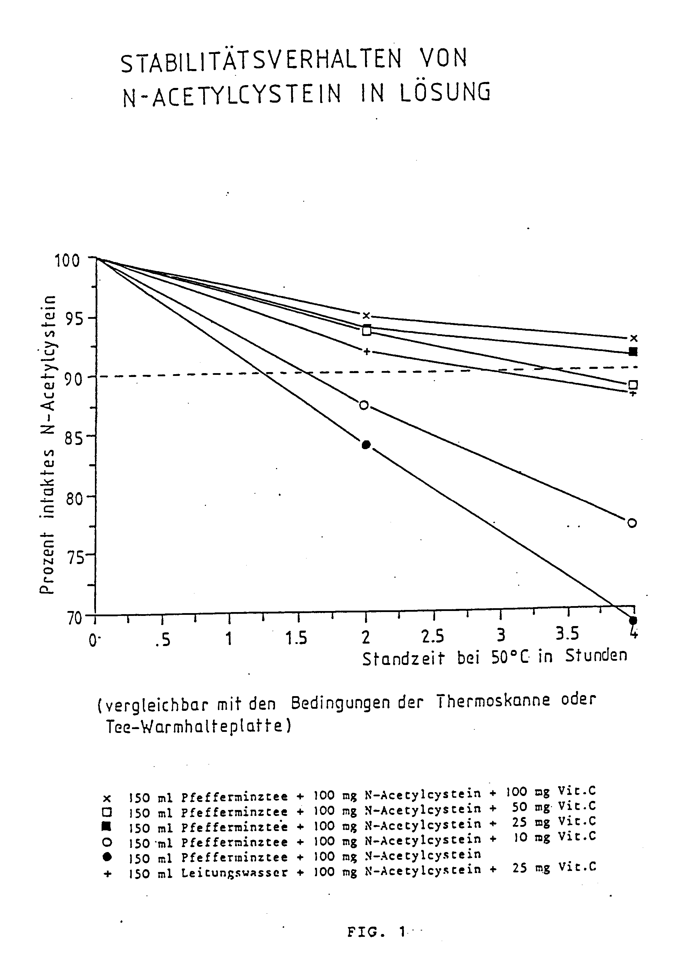

Die Ergebnisse gehen aus den Fig. 1 bis 3 hervor.

Fig. 1 betrifft die stabilisierende Wirkung von Ascorbinsäure auf N-Acetylcystein in Tee (Pfefferminztee) und Leitungswasser von 50°C. Der Kurvenverlauf gibt deutlich wieder, dass in Abwesenheit von Ascorbinsäure nach 2 h bei 50°C mehr als 15 % des Wirkstoffes zerfallen sind. Durch Zugabe von Ascorbinsäure in einer Menge von 25 bis 100 mg kann dieser Zerfall nennenswert reduziert werden. Das gleiche trifft auch für die Anwesenheit von Ascorbinsäure zu, wenn das N-Acetylcystein in Leitungswasser bei 50°C aufgelöst vorliegt.

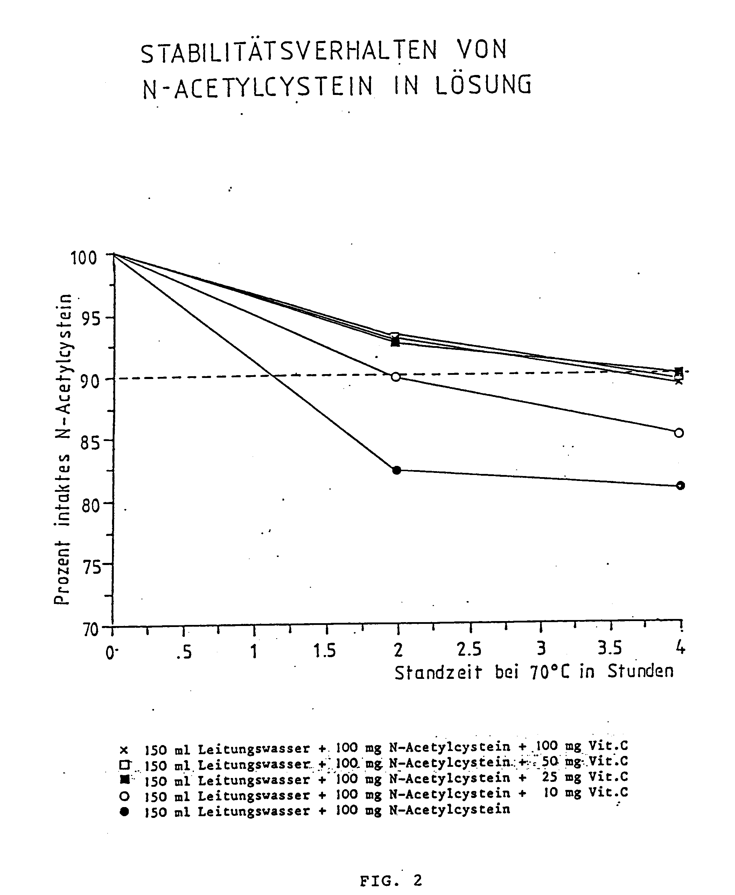

Fig. 2 zeigt die Stabilitätskurven ähnlich wie im Falle von Fig. 1 bei 70°C. In Wasser ist in Abwesenheit von Ascorbinsäure ein nennenswerter Zerfall der Wirkstoffes bei erhöhter Temperatur (70°C) zu beobachten, der durch Zugabe von Ascorbinsäure (bevorzugt 25 bis 50 mg) in seinem Ausmass vermindert ist.

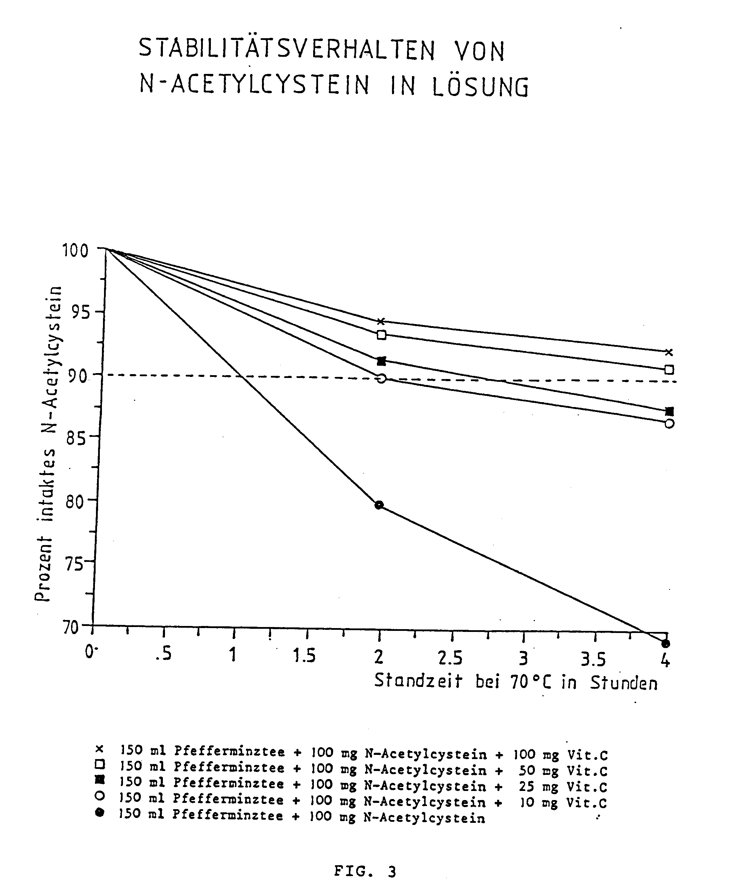

Fig. 3 entspricht den Versuchsbedingungen, wie sie den Versuchen zu Fig. 2 zugrundelagen, doch wurde hier das N-Acetylcystein in Tee (Pfefferminztee) aufgelöst. Auch hier kann durch den stabilisierenden Zusatz von Ascorbinsäure (bevorzugt 25 bis 50 mg) die Inaktivierung des Wirkstoffes reduziert werden.

**Patentansprüche**

**Patentansprüche für folgende Vertragsstaaten : AT, BE, CH, DE, FR, GB, IT, LI, LU, NL, SE**

1. Stabilisiertes N-Acetylcystein-haltiges Arzneimittel, dadurch **gekennzeichnet**, dass es eine therapeutische wirksame Menge an N-Acetylcystein und als alleinigen Stabilisator Ascorbinsäure bzw. Ascorbat umfasst.

2. Arzneimittel nach Anspruch 1, dadurch **gekennzeichnet**, dass dieses in fester Form oder als wässrige Lösung oder Dispersion vorliegt.

3. Arzneimittel nach Anspruch 1 oder 2, dadurch **gekennzeichnet**, dass dieses zur Verabreichung am Menschen in wässrige Lösung gebracht wird.

4. Arzneimittel nach Anspruch 1 bis 3, dadurch **gekennzeichnet**, dass die Ascorbinsäure bzw. das Ascorbat in einem Gewichtsverhältnis von 5 bis 100 Teilen pro 100 Teile N-Acetylcystein vorliegt.

5. Arzneimittel nach Anspruch 4, dadurch **gekennzeichnet**, dass die Ascorbinsäure bzw. das Ascorbat in einem Gewichtsverhältnis von 10 bis 50 Teile pro 100 Teile N-Acetylcystein vorliegt.

6. Arzneimittel nach Anspruch 4, dadurch **gekennzeichnet**, dass die Ascorbinsäure bzw. das Ascorbat in einem Gewichtsverhältnis von 25 Teilen pro 100 Teile Acetylcystein vorliegt.

7. Verwendung von Ascorbinsäure bzw. Ascorbat zur Stabilisierung von N-Acetylcystein-haltigen Arzneimitteln als alleinigen Stabilisator, wobei N-Acetylcystein in einer therapeutisch wirksamen Menge vorliegt.

8. Verwendung nach Anspruch 7, wobei das Arzneimittel als Feststoff, wässrige Lösung oder Dispersion vorliegt.

**Patentansprüche für folgende Vertragsstaaten: ES, GR**

1. Verfahren zur Herstellung eines stabilisierten N-Acetylcystein-haltigen Arzneimittels, dadurch **gekennzeichnet**, dass man eine therapeutisch wirksame Menge an N-Acetylcystein und als alleinigen Stabilisator Ascorbinsäure bzw. Ascorbat formuliert.

2. Verfahren nach Anspruch 1, dadurch **gekennzeichnet**, dass man das Arzneimittel in fester Form und als wässrige Lösung oder Dispersion formuliert.

3. Verfahren nach Anspruch 1 oder 2, dadurch **gekennzeichnet**, dass das Arzneimittel zur Verabreichung am Menschen in wässrige Lösung gebracht wird.

4. Verfahren nach Anspruch 1 bis 3, dadurch **gekennzeichnet**, dass man die Ascorbinsäure bzw. das Ascorbat in einem Gewichtsverhältnis von 5 bis 100 Teilen pro 100 Teile N-Acetylcystein zugibt.

5. Verfahren nach Anspruch 4, dadurch **gekennzeichnet**, dass man die Ascorbinsäure bzw. das Ascorbat in einem Gewichtsverhältnis von 10 bis 50 Teile pro 100 Teile N-Acetylcystein zugibt.

6. Verfahren nach Anspruch 4, dadurch **gekennzeichnet**, dass man die Ascorbinsäure bzw. das Ascorbat in einem Gewichtsverhältnis von 25 Teilen pro 100 Teile Acetylcystein zugibt.

7. Verwendung von Ascorbinsäure bzw. Ascorbat zur Stabilisierung von N-Acetylcystein-haltigen Arzneimitteln als alleinigen Stabilisator, wobei N-Acetylcystein in einer therapeutisch wirksamen Menge vorliegt.

8. Verwendung nach Anspruch 7, wobei das Arzneimittel als Feststoff, wässrige Lösung oder Dispersion vorliegt.

**Claims**

**Claims for the following Contracting States: AT, BE, CH, DE, FR, GB, IT, LI, LU, NL, SE**

1. A stabilized pharmaceutical containing N-acetyl cysteine, characterised in that it comprises a therapeutically effective quantity of N-acetyl cysteine and, as sole stabilizer, ascorbic acid or ascorbate.

2. A pharmaceutical as claimed in Claim 1, characterised in that the said pharmaceutical is present in solid form or in the form of an aqueous solution or dispersion.

3. A pharmaceutical as claimed in Claims 1 or 2, characterised in that the said pharmaceutical is brought into the form of an aqueous solution for administration to humans.

4. A pharmaceutical as claimed in Claims 1 to 3, characterised in that the ascorbic acid or ascorbate is present in a weight ratio of 5 to 100 parts per 100 parts N-acetyl cysteine.

5. A pharmaceutical as claimed in Claim 4, characterised in that the ascorbic acid or ascorbate is present in a weight ratio of 10 to 50 parts per 100 parts N-acetyl cysteine.

6. A pharmaceutical as claimed in Claim 4, characterised in that the ascorbic acid or ascorbate is present in a weight ratio of 25 parts per 100 parts acetyl cysteine.

7. The use of ascorbic acid or ascorbate as sole stabilizer for the stabilization of pharmaceuticals containing N-acetyl cysteine, wherein N-acetyl cysteine is present in a therapeutically effective quantity.

8. The use as claimed in Claim 7, wherein the pharmaceutical is present in the form of a solid, an aqueous solution or a dispersion.

**Claims for the following Contracting State: ES, GR**

1. A process for the production of a stabilized pharmaceutical containing N-acetyl cysteine, characterised in that a therapeutically effective quantity of N-acetyl cysteine and, as sole stabilizer, ascorbic acid or ascorbate are formulated.

2. A process as claimed in Claim 1, characterised in that the pharmaceutical is formulated in solid form and in the form of an aqueous solution or dispersion.

3. A process as claimed in Claim 1 or 2, characterised in that the pharmaceutical is brought into the form of an aqueous solution for administration to humans.

4. A process as claimed in Claims 1 to 3, characterised in that the ascorbic acid or ascorbate is added in a weight ratio of 5 to 100 parts per 100 parts N-acetyl cysteine.

5. A process as claimed in Claim 4, characterised in that the ascorbic acid or ascorbate is added in a weight ratio of 10 to 50 parts per 100 parts N-acetyl cysteine.

6. A process as claimed in Claim 4, characterised in that the ascorbic acid or ascorbate is added in a weight ratio of 25 parts per 100 parts acetyl cysteine.

7. The use of ascorbic acid or ascorbate as sole stabilizer for the stabilization of pharmaceuticals containing N-acetyl cysteine, wherein N-acetyl cysteine is present in a therapeutically effective quantity.

8. Use as claimed in Claim 7, wherein the pharmaceutical is present in the form of a solid, an aqueous solution or a dispersion.


**Revendications**

**Revendications pour les Etats contractants suivants : AT, BE, CH, DE, FR, GB, IT, LI, LU, NL, SE**

1 - Médicament contenant de la N-acétylcystéine stabilisée, caractérisé en ce qu'il comprend une quantité thérapeutiquement active de N-acétylcystéine et, comme seul stabilisant, de l'acide ascorbique ou un ascorbate.

2 - Médicament selon la revendication 1, caractérisé en ce qu'il se présente sous forme solide, de solution ou de dispersion aqueuse.

3 - Médicament selon les revendications 1 ou 2, caractérisé en ce que pour l'administration à l'homme il est amené sous forme de solution aqueuse.

4 - Médicament selon les revendications 1 à 3, caractérisé en ce que l'acide ascorbique ou l'ascorbate sont présents dans un rapport pondéral de 5 à 100 parties pour 100 parties de N-acétylcystéine.

5 - Médicament selon la revendication 4, caractérisé en ce que l'acide ascorbique ou l'ascorbate sont présents dans un rapport pondéral de 10 à 50 parties pour 100 parties de N-acétylcystéine.

6 - Médicament selon la revendication 4, caractérisé en ce que l'acide ascorbique ou l'ascorbate sont présents dans un rapport pondéral de 25 parties pour 100 parties d'acétylcystéine.

7 - Utilisation de l'acide ascorbique ou de l'ascorbate pour la stabilisation de médicaments contenant de la N-acétylcystéine comme seul stabilisant, la N-acétylcystéine étant présente dans une quantité thérapeutiquement active.

8 - Utilisation selon la revendication 7, dans laquelle le médicament est présent sous forme de substance solide, de solution ou de dispersion aqueuse.

**Revendication pour les Etats contractants suivants: ES, GR**

1 - Procédé de préparation d'un médicament contenant de la N-acétylcystéine stabilisée, caractérisé en ce qu'on formule une quantité thérapeutiquement active de N-acétylcystéine, et d'acide ascorbique ou d'ascorbate comme seul stabilisant.

2 - Procédé selon la revendication 1, caractérisé en ce qu'on formule le médicament sous forme solide et sous forme de solution ou de dispersion aqueuse.

3 - Procédé selon les revendications 1 ou 2, caractérisé en ce que pour l'administration à l'homme, on amène le médicament sous forme de solution aqueuse.

4 - Procédé selon les revendications 1 à 3, caractérisé en ce qu'on ajoute l'acide ascorbique ou l'ascorbate dans un rapport pondéral de 5 à 100 parties pour 100 parties de N-acétylcystéine.

5 - Procédé selon la revendication 4, caractérisé en ce qu'on ajoute l'acide ascorbique ou l'ascorbate dans un rapport pondéral de 10 à 50 parties pour 100 parties de N-acétylcystéine.

6 - Procédé selon la revendication 4, caractérisé en ce qu'on ajoute l'acide ascorbique ou l'ascorbate dans un rapport pondéral de 25 parties pour 100 parties d'acétylcystéine.

7 - Utilisation d'acide ascorbique ou d'ascorbate pour la stabilisation de médicaments contenant de la N-acétylcystéine comme stabilisant unique, la N-acétylcystéine étant présente dans une quantité thérapeutiquement active.

8 - Utilisation selon la revendication 7, dans laquelle le médicament est présent sous forme de substance solide, de solution ou de dispersion aqueuse.

# STABILITÄTSVERHALTEN VON
# N-ACETYLCYSTEIN IN LÖSUNG

(vergleichbar mit den Bedingungen der Thermoskanne oder Tee-Warmhalteplatte)

| | |
|---|---|
| ✕ | 150 ml Pfefferminztee + 100 mg N-Acetylcystein + 100 mg Vit.C |
| ☐ | 150 ml Pfefferminztee + 100 mg N-Acetylcystein + 50 mg Vit.C |
| ■ | 150 ml Pfefferminztee + 100 mg N-Acetylcystein + 25 mg Vit.C |
| ○ | 150 ml Pfefferminztee + 100 mg N-Acetylcystein + 10 mg Vit.C |
| ● | 150 ml Pfefferminztee + 100 mg N-Acetylcystein |
| + | 150 ml Leitungswasser + 100 mg N-Acetylcystein + 25 mg Vit.C |

FIG. 1

# STABILITÄTSVERHALTEN VON N-ACETYLCYSTEIN IN LÖSUNG

× 150 ml Leitungswasser + 100 mg N-Acetylcystein + 100 mg Vit.C
□ 150 ml Leitungswasser + 100 mg N-Acetylcystein + 50 mg Vit.C
■ 150 ml Leitungswasser + 100 mg N-Acetylcystein + 25 mg Vit.C
○ 150 ml Leitungswasser + 100 mg N-Acetylcystein + 10 mg Vit.C
● 150 ml Leitungswasser + 100 mg N-Acetylcystein

FIG. 2

# STABILITÄTSVERHALTEN VON N-ACETYLCYSTEIN IN LÖSUNG

X    150 ml Pfefferminztee + 100 mg N-Acetylcystein + 100 mg Vit.C
□    150 ml Pfefferminztee + 100 mg N-Acetylcystein +  50 mg Vit.C
■    150 ml Pfefferminztee + 100 mg N-Acetylcystein +  25 mg Vit.C
O    150 ml Pfefferminztee + 100 mg N-Acetylcystein +  10 mg Vit.C
●    150 ml Pfefferminztee + 100 mg N-Acetylcystein

FIG. 3